# NEW EUROPEAN PATENT SPECIFICATION

(11) **EP 0 530 323 B2**
(45) Date of publication and mention of the opposition decision: **22.12.2004**
(45) Mention of the grant of the patent: 13.01.1999
(21) Application number: 91913303.3
(22) Date of filing: 17.05.1991
(51) Int. Cl.: A61F 2/36

(54) **FEMORAL STEM PROSTHESIS**
FEMURSCHAFTPROTHESE
PROTHESE DE TIGE FEMORALE

(30) Priority: 23.05.1990 US 527298
(43) Date of publication of application: 10.03.1993
(73) Proprietor: Mikhail, Michael W.E., Naples Florida 34103 (US)
(72) Inventor: MIKHAIL, W., E., Michael, Toledo, OH 43623 (US); ELTING, James, J., Oneonta, NY 13820 (US); LING, Robin, S., M., Exeter EX2 4LE (GB)
(74) Representative: West, Alan Harry
(86) International application number: PCT/US1991/003501
(87) International publication number: WO 1991/018561

(56) References cited:
- EP-A- 0 112 423
- EP-A- 0 315 283
- WO-A-91/03992
- FR-A- 2 501 998
- FR-A- 2 575 384
- GB-A- 2 104 391
- US-A- 3 829 904
- US-A- 4 044 403
- US-A- 4 179 758
- US-A- 4 642 124
- US-A- 4 808 186
- US-A- 4 865 608
- US-A- 4 994 085
- NUCLEAR INSTUMENTATION & METHODS IN PHYSICS vol. 50, no. 1/4 , 3 April 1990 , AMSTERDAM NL pages 57 - 61 W. NEUMANN ET AL. 'THIN-LAYER ACTIVATION OF HIP-JOINT PROSTHESES FOR TRIBOLOGICAL TESTS'
- "Experience with the exeter TOMZ Flower et al Hip replacement since 1970", Orthopedic Clinics of North America, Vol. 19, No. 3, July 1988, pp. 477-490, see entire document.
- "Localised Endosteal Bone Lysis in Relation to the Femoral Components of Cemented Total Hip Arthroplasties", P.P. Antony et al., The Journal of Bone and Joint Surgery, Vol. 72-3, No. 6, November 1990, pages 971 to 979
- "1984 Annual Product Catalog", Howmedica Orthopaedics Division, 5 pages of which pages A-1 and A-34 (The Exeter 30 mm Total Hip System
- "Experience with the Exeter Total Hip Replacement Since 1970", J. L. Fowler, A. J. C. Lee and R. S. M. Ling, Orthopedic Clinics of North America, Vol. 19, No. 3, July 1988, pp. 477-489

## Description

The present invention relates to a hip joint prosthesis and more particularly to the femoral component of such a prosthesis.

Many methods and devices have been developed to improve the fixation of hip joint prostheses including the femoral component thereof in the body so that the device implanted therein becomes as permanent as possible. Many orthopedic implants use a cement to anchor the stem portion of a femoral component in the femur. For example, United Kingdom Patent Specification No. 1,409,054 discloses a hip joint prosthesis having a double-tapered stem which, among other advantages, enhances extrusion of cement caused by penetration of the stem during fixation. U.S. Patent No. 3,793,650 discloses an intramedullary stem for a prosthetic bone joint device having a base with spring members intended to centralize the position of the stem in the canal or bore of the bone in order to insure a relatively uniform or, at least minimum thickness of cement between the wall of the bone and the stem. It is desirable that there be at least two millimeters (2mm) of cement between the stem and the bone. By providing a means for insuring that there will be at least a certain minimum thickness of cement between the stem of the prosthesis and the interior wall of the canal formed in the femur bone for receiving such stem, the likelihood of the stem protruding through the cement and contacting the interior of the femur bone itself is minimized. Thus, in those types of implants using cement, it is important to insure that the stem is completely encapsulated by the cement and does not protrude through to contact the bone.

One type of bone cement utilized to retain the stem of a femoral hip joint prosthesis in the canal of a bone comprises a mixture of polymethylmethacrylate (hereinafter PMMA) polymer and methyl methacrylate monomer and optionally including a styrene copolymer of PMMA. This and other types of cement utilized for such purpose may be packaged in two separate components which are mixed into a paste which is placed in the canal of the femur immediately prior to insertion of the stem of the prosthesis. Such paste then sets to a relatively rigid material providing excellent adherence to the interior wall of the bone.

Heretofore, it has been the belief that it is desirable to have good adhesion between the stem and the cement. Many prior art devices were specifically directed to providing a design for the prosthesis intended to maximize adhesion between it and the cement. For example, the CML Cemented Medullary Locking Hip System manufactured by DePuy Division of Boehringer Mannheim Corporation, Warsaw, Indiana, is a hip system in which the upper portion of the stem is provided with a roughened textured surface intended to enhance the bond of the cement to the prosthesis at the prosthetic interface. It also utilizes a "Macro-Textured" waffle design which is intended to increase the surface area and the mechanical interlock between the cement and the prothesis in the area of such waffle design.

Osteonics Corp., Allendale, N.J., manufactures the OMNIFLEX Femoral System of a titanium alloy having a normalized surface to promote good adhesion of the cement thereto.

U.S. Patent No. 4,281,420 is directed to maximizing the strength and durability of the prosthesis/cement adherence.

Other types of devices which disclose the use of cement within a bore or canal of the femur are described in U.S. Patent Nos. 3,829,904; 3,874,003; 4,012,796; and 4,080,666.

EP 0 315 283 describes a method of shaping an endoprosthesis, a femoral head prosthesis, an acetabulum prosthesis and a method of fixing a femoral head prosthesis in bone.

Still other types of prostheses are intended for use without cement. Many of these are designed to provide a porous or roughened surface in order that the bone may grow into the porous surface of the prosthesis. For example, U.S. Patent No. 3,808,606 discloses a prosthesis possessing porous surfaces for fixation by tissue ingrowth. U.S. Patent No. 4,164,794 discloses prosthetic devices having outer foamed or sintered porous coatings of selected bioenegineered thermoplastics which enables the device to become firmly anchored to the bone by tissue ingrown into the coated material.

In both the cemented and non-cemented types of devices use heretofore, problems have arisen, particularly after a number of years of implantation. With respect to the cemented type devices part of the problem arises from the fact that the cement utilized to retain the stem of the device in the canal of the femur bone is subject to a phenomenon known as creep. Thus. while the bone cement appears to be rigid when set, it is subject to minute amounts of movement over time. The amount of creep encountered with such cement following implantation is exaggerated by virtue of the fact that the body temperature controls the temperature of the implanted cement and prosthesis. Thus PMMA and other types of bone cement at body temperature are subject to a greater degree of creep than bone cement maintained at room temperature of, say, 22°C (72°F). This may be readily observed by mounting a bar of PMMA so that its ends are supported and applying a fixed load at the center of the bar. Tests have shown that a bar so supported and subjected to a load of 5 pounds for eight hours at 37°C (98.6°F) will deflect to an extent 3.5 times greater than an identical bar supported and loaded in an identical manner for eight hours at 22°C(72°F).

Over a period of time, the phenomenon of creep may result in disruption over the micro-interlocking of the cement-bone interface especially if the cement mantle is firmly bonded to the femoral prosthesis. As is well known in the field of hip replacements, it is important that there be a good bond between the cement and the bone and that there be no disruption in the micro-interlocking of the cement-bone interface.

Subsidence of the femoral component occurs in various degrees with prostheses of different designs regardless of the presence or absence of collars. Any firmly bonded or fixed prosthesis to the cement will disrupt the cement bone interface which will inevitably lead to clinical loosening and subsequent failure necessitating revision.

According to the present invention there is provided a collarless femoral hip joint prosthesis adapted to be cemented into an intramedullary canal, the prosthesis having an elongated stem which has a proximal end and a distal end extending along a first axis (C), the stem being convergently tapered towards its distal end and having its proximal end extending to an area of juncture with a neck portion extending along a second axis (D) disposed at an obtuse angle to the first axis, the area of juncture forming a smooth arcuate contour in the included angle between the first and second axes and an enlarged shoulder on the opposite side, the shoulder including a lower portion which follows a straight line path aligned with the stem and an upper portion which follows a smooth curved path merging with the neck portion and a straight line path from one edge to the opposite edge across its width, the area of juncture between the lower portion and the upper portion providing a line of demarcation at the outermost portion of the shoulder, the stem having a circular cross-sectional configuration near its distal end and an oval cross-sectional configuration in the area approaching the arcuate contour and the shoulder, the cross-sectional configurations in intermediate areas merging process between circular and oval, the prosthesis being formed of cobalt-chromium-molybdenum alloy and the stem having a smooth polished surface with a surface roughness equal to or less than 102 nm (4 microinches).

The present invention provides for a femoral hip joint prosthesis having a design which allows for subsidence of the stem within the cement mantle without disrupting the micro-interlocking in the cement-bone interfaces.

Accordingly, it is an object of the present invention to provide a new and novel femoral hip joint prosthesis which is specifically designed to avoid the aforementioned problems resulting from subsidence of the stem and physical properties of the cement at different temperatures.

It is a further object of the present invention to provide a femoral hip joint prosthesis which will not loosen but rather will self tighten even though the cement mantle creeps or expands fractionally over a period of time.

It is yet another object of the present invention or provide a femoral hip joint prosthesis in which the stem subsides within the cement as the cement creeps and, thus, is permitted to remain at all times in snug interfacial contact therewith, imparting in the stem area the reliable compressive forces against the cement which is micro-interlocked with the bony surfaces.

It is another object of the present invention to provide a femoral hip joint prosthesis which self compensates and subsides within a cement mantle as such cement mantle creeps over time without disrupting the micro-interlocking and thus preserving the cement-bone interface.

The femoral hip joint prosthesis of the present invention is collarless, has a double tapered stem formed of cobalt chrome molybdenum alloy and has the surface of the stem highly polished to provide an extremely smooth surface which should not exceed maximum level of roughness. The lower end of the stem is positioned in a hollow centralizer which serves to stabilize it and insure that an adequate thickness of cement encapsulates the stem. Such design permits the stem portion of the prosthesis to move fractionally within the cement mantle without disrupting the cement-bone interface and to self-tighten as the male component, namely, the distal tip of the stem engages further in the hollow centralizer.

Although prior art prostheses such as the tapered collarless bone joint devices disclosed in the previously referenced United Kingdom Patent Specification No. 1,409,054 and U.S. Patent No. 3,793,650 have been used with polished surfaces, they have never utilized chrome cobalt alloy with the roughness tolerance as set forth in the present invention. The prosthesis of the present invention provides superior results over the prior art in that, as well as allowing subsidence within the cement mantle, it exhibits good corrosion resistance when implanted in the body. The absence of a collar is important to provide a prosthesis in which there is no mechanical interference with any such subsidence. The combination of medial cement buttress and absence of a collar prevent the stem from going into varus when subsidence occurs.

Additionally, the femoral hip joint prosthesis of the present invention at the proximal end opposite the stem is contoured to provde a smooth transation to a Morse taper which may receive Morse Taper Heads of varying neck lengths and diameters (i.e. 22, 26, 28, 32mm).

Finally, it is an object of the present invention to provide a femoral hip joint prosthesis in combination with a cement mantle implanted in the canal of a femur wherein said cement mantle encapsulates the stem of such prosthesis in an interfacial relationship which permits subsidence of the stem within the cement mantle without disrupting the interfacial adherance between the cement mantle and the bone.

### BRIEF DESCRIPTION OF THE DRAWINGS

Fig. 1 is a front elevational view of a femoral hip joint prosthesis;
Fig. 2 is an end view of such femoral hip joint prosthesis;
Fig. 3 is a sectional view taken through line 3-3 of Fig. 1;
Fig. 4 is a sectional view taken through line 4-4 of Fig. 1;
Figs. 5 and 6 are schematic views showing the difference in the amount of deflection occurring in a test bar of PMMA bone cement supported at its ends and subjected to a constant load of 5 pounds for eight hours at room temperature (Fig. 5) and at a temperature approximating body temperature (Fig. 6);
Fig. 7 is a sectional view showing a femoral hip joint prosthesis immediately after implanting in a patient;
Fig. 8 is a view similar to Fig. 7 showing the femoral hip joint prosthesis after being implanted for a number of years and showing, greatly exaggerated, the effects of subsidence;
Fig. 9 is a view similar to Fig. 8 showing the difference in the effect of the subsidence on a femoral hip joint prosthesis of the type in which the cement is rigidly adhered to the femoral hip joint prosthesis.
Fig. 10 is a view of a femoral hip joint prosthesis having a collar after being implanted for a number of years and showing the effect of subsidence on a prosthesis having a collar.
Figs. 11-13 are elevational views showing the femoral hip joint prosthesis of Fig. 1 in combination with Morse Taper Heads of various lengths.
Fig. 14 is a front elevational view of a femoral hip joint prosthesis according to the present invention.
Fig. 15 is an end view of the femoral hip joint prosthesis of Fig. 14.
Fig. 16 is a sectional view taken through line 16-16 of Fig. 14.
Fig. 17 is a sectional view taken through line 17-17 of Fig. 14.
Fig. 18 is a sectional view taken through line 18-18 of Fig. 14.

### Detailed Description Of The Drawings

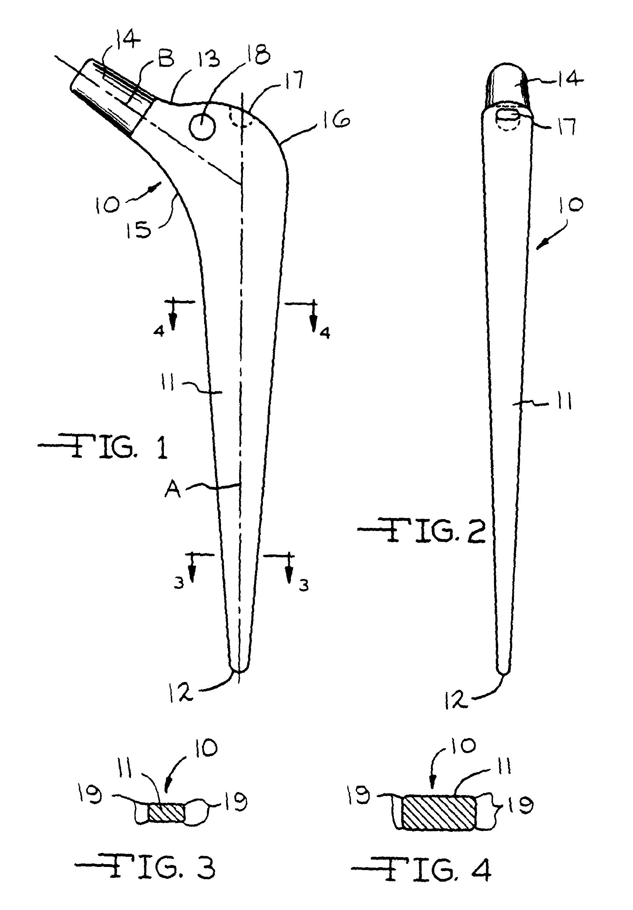

Referring now to Figs. 1 and 2, there is shown a femoral hip joint prosthesis 10 having a stem 11 which is convergently tapered toward a distal end 12 and extending along a first axis of symmetry A to an area of juncture with a neck portion 13 lying on a second axis of symmetry B. Extending from the neck portion 13 is a frustoconically shaped Morse Taper Neck 14 to which may be attached a spherically shaped Morse Taper Head. As is clear from Fig. 1, no collar is provided in the femoral hip prosthesis, but rather the portion of the prosthesis joining the stem 11 to the neck 13 follows a smooth arcuate contour in the area 15 of the included angle between the respective axes of symmetry A and B. The portion of the femoral hip prosthesis 10 opposite the smooth arcuate portion 15, namely, that portion on the outside of the angle between the two axes of symmetry A and B, has an enlarged shoulder 16 in which is formed a dimple or recess 17 for driving the prosthesis into the femur. As can be seen, the dimple 17 is located on the first axis of symmetry A.

An aperture 18 is provided in the area of the neck and shoulder to assist in removing the prosthesis 10 in the event revision is required at some future time.

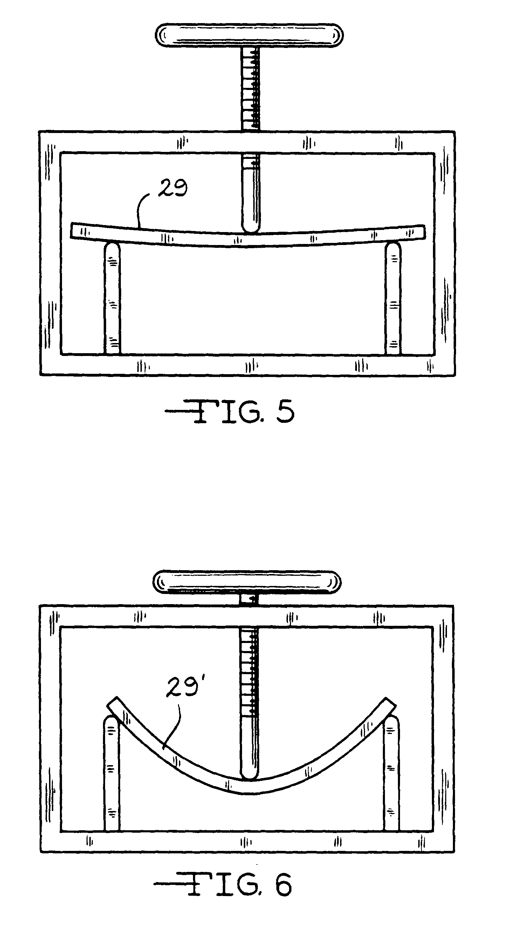

As can be seen in Figs. 3 and 4, the stem 11 is tapered in both directions and has rounded corners 19. As pointed out in United Kingdom Patent Specification 1,409,054, such double tapering enhances the extrusion of cement caused by penetration of the stem 11 thereinto during fixation.

The femoral hip joint prosthesis 10 of the present invention is formed of high-strength forged Co-Cr-Mo alloy (ASTM designation F-799) and has its surface polished to a high degree (also known as a color buff finish) to provide for a smoothness having a target surface roughness of 11nm (four (4) microinches). It has greater fatigue strength, corrosion resistance and wear resistance than stainless steel. Additionally, it resists pitting and crevice corrosion in the body environment.

It is the combination of the Co-Cr-Mo alloy having its surface polished to the above target coupled with the tapered stem and collarless design which permits the femoral hip prosthesis to function in the manner intended without loosening and without causing pain or other adverse mechanical effects in the patient even though there is subsidence of the prosthesis over a period of time. Thus, the present design permits the polished stem to subside within the cement mantle. The taper of the stem permits it to self-tighten upon the slight movement which occurs during the subsidence and engage in the hollow centralizer and yet to do so without pulling the cement mantle and thus avoid disrupting the micro-interlocking at the cement-bone interface. Such design causes the stem to impart primarily compressive forces against the cement mantle, thus transmitting the load to the femur. Transmitting the load in this manner forces the cement mantle continuously snugly and firmly against the interior of the femur to assist in maintaining the integrity of the micro-interlocking at the cement-bone interface.

Referring now to Fig. 5, a bar 29 of PMMA cement 80mm long and having a rectangular cross section of 10mm by 3mm was supported on point supports 75mm apart and subjected to a load at its midpoint of 3.9 kg. while being maintained at a temperature of 22°C (72°F) for ten hours. Such loading resulted in a deflection of 4.3mm.

Fig. 6 illustrates an identical bar 29' loaded in the same manner except at a temperature of 37°C (98.6°F). The bar 29' had a deflection of 15mm at the end of such ten hour period.

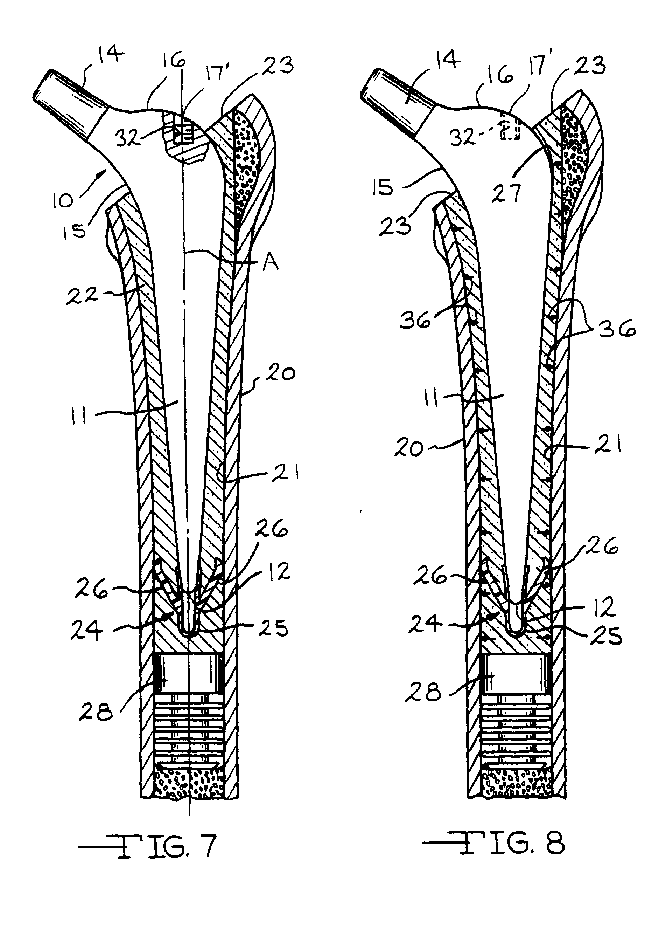

Referring now to Fig. 7, there is shown the femoral hip joint prosthesis 10 immediately following its implantation in the femur bone 20. As is customary, the femur bone 20 is prepared by reaming a canal 21 into which PMMA or other suitable bone cement is introduced under pressure. Promptly after introduction of the bone cement into the canal 21 and before the cement has had an opportunity to set, the stem 11 of the femoral hip joint prosthesis 10 is inserted into the cement with the result that a cement mantle 22 is formed around the stem 11 up to the arcuate area 15 and a portion of the enlarged shoulder 16. Any excess cement is wiped away leaving an exposed upper end 23. The free or distal end 12 of the stem 11 is engaged in a hollow plastic centralizer 24 which insures that there will be a sufficient thickness of cement around all portions of the stem. The plastic centralizer 24 includes a cup-shaped pocket 25 having a plurality, preferrably 3 or 4, of integrally formed resilient arms 26 sized to engage the interior of the canal 21. The hollow cup-shaped pocket 25 of the centralizer may be filled with a compressible material such as Avitin Powder, Surgicell, Gelfoam or the like such that there will be no interference with subsidence of the distal end 12 of the prosthesis 10 within the hollow pocket 25 of the centralizer. Prior to introduction of cement in the canal, a cement restrictor 28 is positioned therein.

Fig. 8 shows the implanted femoral hip joint prosthesis 10 after an extended period, say ten years, following implantation. As can be seen there has occurred a small amount of radiological subsidence, on the average of 2 mm, where the stem 11 has subsided within the cement mantle 22. As may be seen in Fig. 8, such subsidence within the cement mantle results in the distal end 12 moving further into the centralizer 27 and in the enlarged shoulder 16 pulling away from the cement mantle 22 leaving a gap 27. Because of the tapered-stem, collarless design of Co-Cr-Mo alloy having a highly polished surface, the femoral hip joint prosthesis 10 is permitted to subside within the cement mantle 22 but to do without disrupting the cement-bone interface. Thus, the subsidence of the stem 11 results in microscopic movement of the stem 11 in relation to the adjacent surface of the cement mantle 22. As will be appreciated and as shown schematically in Fig. 8, the effect of such microscopic movement is to cause the stem 11 to self-tighten as it and the cement mantle 22 subside and to impart primarily compressive forces against the cement mantle 22 in directions substantially normal to the interior surfaces of the bone 20. This is illustrated schematically by the arrows 36 in Fig. 8.

The hip joint prosthesis 10 shown in Figs. 7 and 8 incorporates a modification in that the dimple 17' is drilled to a deeper depth than the dimple 17 of Figs. 1 and 2 and is tapped to form internal threads 32. The threaded dimple 17' may then serve the dual function of assisting with insertion of the hip joint prosthesis 10 and, in the event replacement is required, with its removal. As can be seen from Figs. 7 and 8, with this modification, no hole is provided in the shoulder 16. As will be appreciated, a tool (not shown) engages the threads 32 to assist in such insertion or removal.

Fig. 9 shows schematically the effect of subsidence of the bone cement 22' on a femoral hip joint prosthesis 10' having a similar configuration as that of femoral hip joint prosthesis 10 but having a porous or other type surface 38 to which the bone cement may become firmly bonded to create an interlocked stem-cement mantle interface. As can be seen from the schematic, the subsidence of the stem 11' to which the cement mantle 22' is firmly bonded, will cause the cement mantle 22' to be pulled downwardly with the stem 11' as it subsides thus placing tension upon the interface between the cement mantle 22' and the bone, causing an undesirable disruption in the micro-interlocking of the bone-cement interface and inevitable subsequent loosening thereof. Such tension on the interface between the cement mantle 22' and the bone 20 is illustrated schematically by the arrows 37 as the stem 11' tends to pull with it the adjacent portions of the cement mantle 22' bonded thereto.

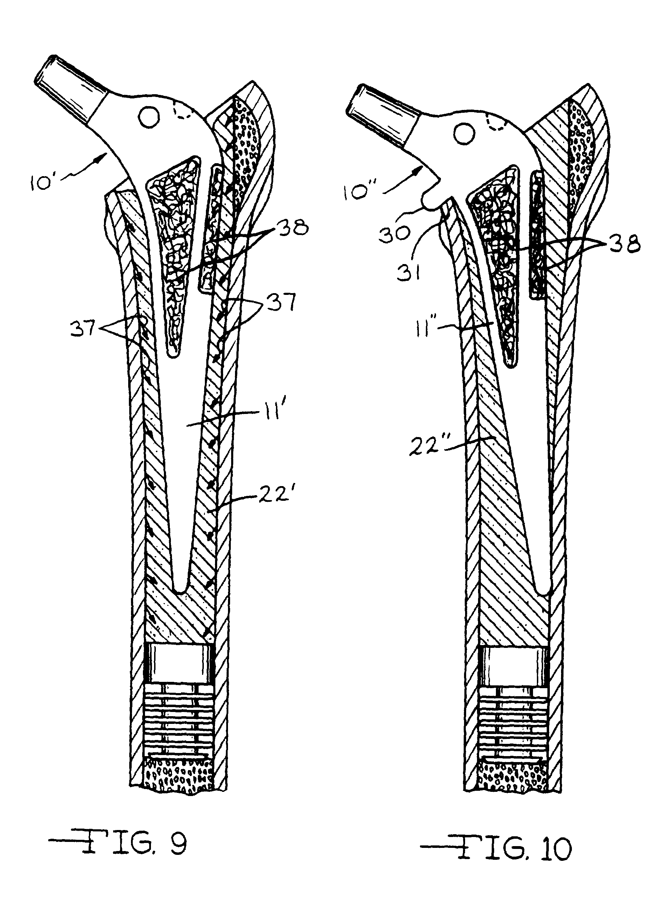

Fig. 10 shows the undesirable effect of subsidence on a femoral hip joint prosthesis 10" having a collar 30. In those types of prostheses having collars such as that illustrated by the collar 30 in Fig. 10, the prosthesis is positioned during implantation with the collar 30 resting upon the top 31 of the prepared femur. As can be seen in Fig. 10, such collar 30 interferes with subsidence of the stem 11" within the cement mantle 22" with the result that the stem 11" becomes cocked in the canal of the femur and may, in extreme cases, be forced through the cement mantle and into contact with the interior of the bone causing great discomfort to the patient. Additionally, the pressure of such collar 30 against the top 31 or calcar of the prepared femur, causes a substantial wearing away of the calcar.

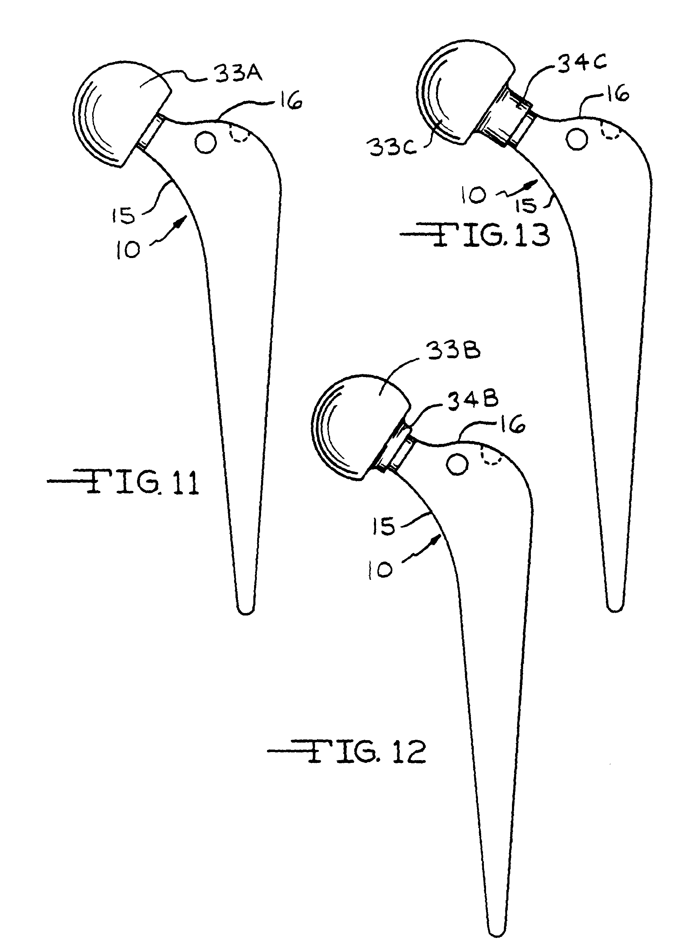

Referring now to Figs. 11-13, there is shown three different Morse Taper Heads 33A (Fig. 11), 33B (Fig. 12) and 33C (Fig. 13). As can be seen from a comparison, Morse Taper Head 33A has no neck, Morse Taper Head 33B has a short neck 34B and Morse Taper Head 33C has a relatively long neck 34C. This permits effective elongation of the stem into the canal. The surgeon will make a determination during implantation of which of the Morse Taper Heads to use with a given patient based upon a number of factors based upon the leg length and stability of the prosthesis. Additionally, the heads 33A, 33B and 33C may be provided with different diameters ranging from 22, 26, 28 to 32mm.

As can be seen from the drawings, the prosthesis 10 follows a smooth contour from the arcuate area 15 on one side and the enlarged shoulder 16 on the other side to the frustoconically shaped portion forming the Morse Tapered Neck 14. The absence of any protrusions in such area is a factor which permits the effective subsidence of the prosthesis 10 within the cement mantle.

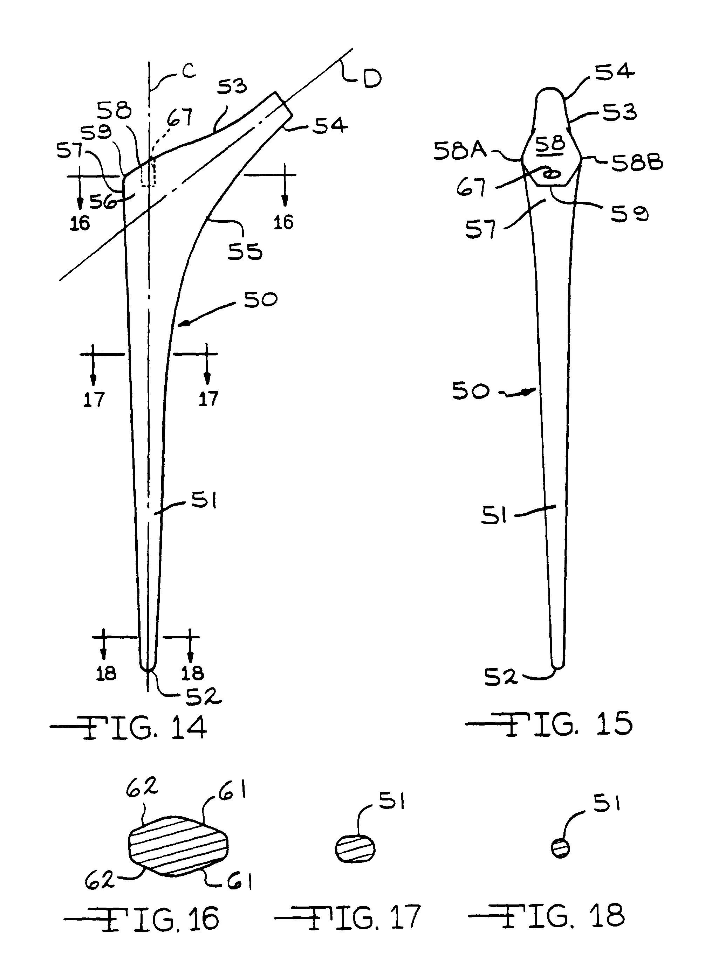

Referring now to Figs. 14-18, there is provided a femoral hip prosthesis 50 according to the present invention, having a stem 51 which is convergently tapered toward the distal end 52 and extending along a first axis of symmetry C to an area of juncture with an neck portion 53 lying on a second axis of symmetry D. Extending from the neck portion 53 is a frustoconically-shaped Morse Taper Neck 54 to which may be attached a spherically-shaped Morse Taper Head. As in the previous embodiments, no collar is provided in the femoral hip prosthesis 50, but rather the portion of the prosthesis joining the stem 51 to the neck 53 follows a smooth arcuate contour in the area 55 of the included angle between the respective axes of symmetry C and D. The portion of the femoral hip prosthesis 50 opposite the smooth arcuate portion 55, namely, that portion on the outside of the angle between the two axes of symmetry C and D has an enlarged shoulder 56. As may be seen in Fig. 14, in profile the shoulder 56 includes a lower portion 57 which follows a straight line path aligned with the straight line path followed by a stem 51 and an upper portion 58 which follows a smooth slightly curved path merging with the neck portion 53.

As may be seen from Fig. 15, the upper portion of the shoulder 58 follows a straight line path from one edge 58A to the opposite edge 58B. The area of juncture between the lower portion 57 and upper portion 58 provides a sharp line of demarcation 59 at the outer most portion of the shoulder 56.

As may be seen in Fig. 14, the stem 51 diverges away from the axis C as it extends from the distal end 52 toward the shoulder 56. As may be seen from Figs. 18 and 17, the stem 51 has a circular cross-sectional configuration near the distal end 52 and an oval configuration in the area approaching the arcuate area 55 and the shoulder 56 with the areas therebetween merging between circular and oval.

Additionally, as may be seen in Fig. 16, the cross-sectional configuration in the area through the neck 53 and upper portion 58 of the shoulder 56 has a generally oval configuration with flattened segments 61 and 62 on opposite sides. Other cross-sectional configurations may be utilized in this area provided it merges smoothly with the stem 51 and the neck 53.

As in the previous illustration the upper portion 58 of the shoulder 56 may have formed therein a dimple or recess 67 which may, if desired, have internal threads (not shown). As in the previous illustration, the dimple 67 is located on the first axis of symmetry C.

The present invention of a femoral hip joint prosthesis, formed of Co-Cr-Mo alloy with a highly polished surface, collarless and a tapered stem as set out in the claim hereinbelow, permits patients to enjoy long lasting and predictable results.

Modifications will be readily apparent to those skilled in the art. Accordingly, the present invention should be limited only by the scope of the claims.

## Claims

1. A collarless femoral hip joint prosthesis (50) adapted to be cemented into an intramedullary canal, the prosthesis having an elongated stem (51) which has a proximal end and a distal end (52) extending along a first axis (C), the stem being convergently tapered towards its distal end and having its proximal end extending to an area of juncture with a neck portion (53) extending along a second axis (D) disposed at an obtuse angle to the first axis, the area of juncture forming a smooth arcuate contour (55) in the included angle between the first and second axes and an enlarged shoulder (56) on the opposite side, the shoulder including a lower portion (57) which follows a straight line path aligned with the stem and an upper portion (58) which follows a smooth curved path merging with the neck portion and a straight line path from one edge (58A) to the opposite edge (58B) across its width, the area of juncture between the lower portion and the upper portion providing a line of demarcation (59) at the outermost portion of the shoulder, the stem having a circular cross-sectional configuration near its distal end (52) and an oval cross-sectional configuration in the area approaching the arcuate contour (55) and the shoulder (56), the cross-sectional configurations in intermediate areas merging between circular and oval the prosthesis being formed of cobalt-chromium-molybdenum alloy and the stem having a smooth polished surface with a surface roughness equal to or less than 102 nm (4 microinches).

## Patentansprüche

1. Eine kragenlose femorale Hüftgelenksprothese (50), die dafür ausgelegt ist, in einen intramedullären Kanal einzementiert zu werden, wobei die Prothese einen langgestreckten Schaft (51) hat, der ein proximales Ende und ein distales Ende (52) aufweist, das sich entlang einer ersten Achse (C) erstreckt, wobei sich der Schaft konvergierend in Richtung des distalen Endes verjüngt und sich sein proximales Ende in einen Verbindungsbereich mit einem Halsabschnitt (53) erstreckt, der entlang einer zweiten Achse (D) verläuft, die in einem stumpfen Winkel zu der ersten Achse angeordnet ist, wobei der Verbindungsbereich eine glatte bogenförmige Kontur (55) in dem eingeschlossenen Winkel zwischen den ersten und zweiten Achsen und eine vergrößerte Schulter (56) an der gegenüberliegenden Seite bildet, wobei die Schulter einen unteren Abschnitt (57), der einem geradlinigen Pfad folgt, der mit dem Schaft in Fluchtung ist und einen oberen Abschnitt (58) hat, der einem glatten gekrümmten Pfad folgt, der in den Halsabschnitt übergeht und einen geradlinigen Pfad von einer Kante (58A) zur gegenüberliegenden Kante (58B) über ihre Breite hinweg hat, wobei der Verbindungsbereich zwischen dem unteren Abschnitt und dem oberen Abschnitt eine Grenzlinie (59) am äußersten Abschnitt der Schulter schafft, wobei der Schaft nahe seines distalen Endes (52) kreisförmige Querschnittsform und ovale Querschnittsform in einem Bereich hat, der sich der bogenförmigen Kontur (55) und der Schulter (56) annähert, wobei die Querschnittsformgebungen an dazwischen liegenden Bereichen von kreisförmig in oval übergehen, wobei die Prothese aus einer Kobald-Chrom-Molybdän-Legierung gebildet ist und der Schaft eine glatte polierte Oberfläche mit einer Oberflächenrauhigkeit gleich oder geringer als 102 nm (4 Mikroinch) hat.

## Revendications

1. Prothèse fémorale sans bride (50) pour la hanche, apte à être placée dans un canal intramédulaire, comportant une tige allongée (51) avec une extrémité proximale et une extrémité distale (52) s'étendant le long d'un premier axe (C), ladite tige allant en diminuant vers son extrémité distale tandis que son extrémité proximale s'étend vers une zone de jonction avec une partie formant col (53) le long d'un second axe (D) disposé suivant un angle obtus par rapport au premier axe, la zone de jonction formant un contour arqué lisse (55) dans l'angle défini entre les premier et second axes, et un épaulement élargi (56) sur le côté opposé, lequel épaulement comprend une partie inférieure (57) qui décrit une ligne droite dans l'alignement de la tige, et une partie supérieure (58) qui décrit une courbe lisse se prolongeant par la partie formant col, et une ligne droite d'un bord (58A) au bord opposé (58B) sur sa largeur, la zone de jonction entre les parties inférieure et supérieure définissant une ligne de démarcation (59) sur la partie extérieure de l'épaulement, la tige ayant une forme de section transversale circulaire près de son extrémité distale (52), et une forme de section transversale ovale dans la zone voisine du contour arqué (55) et de l'épaulement (56), la section transversale des zones intermédiaires passant d'une forme circulaire à une forme ovale, la prothèse se composant d'un alliage cobalt-chrome-molybdène et présentant une surface polie lisse dont la rugosité est inférieure ou égale à 102 nm (4 micropouces).
